# EUROPÄISCHE PATENTANMELDUNG

(11) **EP 4 809 970 A1**
(43) Veröffentlichungstag der Anmeldung: **23.09.2026**
(21) Anmeldenummer: 25165374.7
(22) Anmeldetag: 21.03.2025
(51) Int. Cl.: A61C 7/08, A61F 5/56, A63B 71/08

(54) **VERFAHREN ZUM HERSTELLEN EINER ZAHNSCHIENE, ZAHNSCHIENE, ZAHNSCHIENENBAUGRUPPE, UND FOLIENMATERIAL ZUM HERSTELLEN EINER ZAHNSCHIENE**

(71) Anmelder: Ivoclar Vivadent AG, 9494 Schaan (LI)
(72) Erfinder: SKAWRAN, Alexander, 7320 Sargans (CH); SCHÜLKE, Andreas, 9472 Grabs (CH)
(74) Vertreter: Maiwald GmbH

(57) **Zusammenfassung**

Es wird ein Verfahren zum Herstellen einer Zahnschiene (10) beschrieben. Das Verfahren umfasst ein Bereitstellen einer zahnmedizinischen Funktionsfolie (14), wobei in der Funktionsfolie (14) eine Mehrzahl an Durchgangsöffnungen (16) für Speichel und/oder Nahrungspartikel vorhanden ist. Ferner umfasst das Verfahren ein luftdichtes Verschließen der Mehrzahl an Durchgangsöffnungen (16) mittels einer Dichtfolie (24). Außerdem werden die Funktionsfolie (14) und die Dichtfolie (24) gemeinsam entlang eines Zahnmodells (28) tiefgezogen. Anschließend wird die Dichtfolie (28) von der Funktionsfolie (14) abgenommen. Zudem wird eine Zahnschiene vorgestellt, die mittels eines solchen Verfahrens erhalten wird. Ferner wird eine Zahnschienenbaugruppe (30) präsentiert, die einen Zahnschienenkörper (12) umfasst, der aus einer zahnmedizinischen Funktionsfolie (14) gebildet ist, die eine Mehrzahl an Durchgangsöffnungen (16) für Speichel und/oder Nahrungspartikel aufweist, wobei die Durchgangsöffnungen (16) mittels einer Dichtfolie (24) luftdicht verschlossen sind. Ebenso wird ein Folienmaterial (22) zum Herstellen einer Zahnschiene (10) erläutert.

## Beschreibung

Die Erfindung betrifft ein Verfahren zum Herstellen einer Zahnschiene.

Die Erfindung ist ferner auf eine Zahnschiene gerichtet, die mittels eines solchen Verfahrens erhalten wird.

Außerdem betrifft die Erfindung eine Zahnschienenbaugruppe sowie ein Folienmaterial zum Herstellen einer Zahnschiene.

Zahnschienen werden für verschiedene zahnmedizinische Behandlungen verwendet. Beispielsweise werden Zahnschienen dazu verwendet, Zahnstellungen zu korrigieren. In diesem Zusammenhang werden die Zahnschienen auch als Aligner-Schienen bezeichnet. Ein weiterer Anwendungsfall von Zahnschienen ist der Schutz der Zähne und die Entlastung der Kiefergelenke. Umgangssprachlich werden in diesem Zusammenhang die Zahnschienen auch als Knirscher-Schienen bezeichnet. Auch werden Zahnschienen zum Schutz der Zähne bei verschiedenen sportlichen Aktivitäten eingesetzt.

In diesem Zusammenhang ist es ferner bekannt, Zahnschienen mittels Tiefziehverfahren herzustellen. Dabei wird eine Folie, aus der die Zahnschiene gefertigt werden soll, unter Einfluss von Druck, der auch ein Unterdruck sein kann, und Wärme über ein Zahnmodell gezogen, welches meist aus Gips ist. Auch ist es bekannt, ein derartiges Zahnmodell mittels eines 3D-Druckverfahrens herzustellen.

Bei der Herstellung von Zahnschienen muss stets berücksichtigt werden, dass einerseits die Herstellung einfach, zuverlässig und effizient sein soll. Andererseits muss die zahnmedizinische Funktion sichergestellt sein. Dies birgt einen gewissen Zielkonflikt.

Die der vorliegenden Erfindung zugrundeliegende Aufgabe besteht somit darin, diesen Zielkonflikt aufzulösen oder zumindest abzumildern, indem Verfahren zur Herstellung von Zahnschienen derart verbessert werden, dass Zahnschienen mit umfangreicher zahnmedizinischer Funktion einfach, zuverlässig und effizient hergestellt werden können.

Die Aufgabe wird durch ein Verfahren zum Herstellen einer Zahnschiene gelöst. Das Verfahren umfasst
- Bereitstellen einer zahnmedizinischen Funktionsfolie, wobei in der Funktionsfolie eine Mehrzahl an Durchgangsöffnungen für Speichel und/oder Nahrungspartikel vorhanden ist,
- luftdichtes Verschließen der Mehrzahl an Durchgangsöffnungen mittels einer Dichtfolie,
- gemeinsames Tiefziehen der Funktionsfolie und der Dichtfolie entlang eines Zahnmodells, und
- anschließendes Abnehmen der Dichtfolie von der Funktionsfolie.

Die eigentliche Zahnschiene wird somit aus der zahnmedizinischen Funktionsfolie mit den Durchgangsöffnungen für Speichel und/oder Nahrungspartikel hergestellt. Die Zahnschiene hat also mehrere Löcher oder Öffnungen, die so dimensioniert sind, dass ein Speichelaustausch sowie ein Transfer von Nahrungspartikeln von einer Seite auf eine andere Seite der Zahnschiene möglich sind. Derartige Zahnschienen haben mehrere zahnmedizinische Vorteile. Was die Nahrungspartikel betrifft, so ist es mit derartigen Zahnschienen möglich, die Nahrungspartikel durch die Durchgangsöffnungen aus dem Bereich der Zähne abzuführen. Mit anderen Worten wird durch die Durchgangsöffnungen verhindert, dass Nahrungspartikel mittels der Zahnschiene im Bereich der Zähne gehalten werden. Das reduziert die Gefahr für Karies und Gingivitis. Auch der Austausch von Speichel zwischen derjenigen Seite der Zahnschiene, die an den Zähnen anliegt und derjenigen Seite der Zahnschiene, die von den Zähnen weg weist, ist vorteilhaft. Ein derartiger Austausch von Speichel reduziert die Bildung von Zahnbelägen sowie das Risiko von Karies und Gingivitis. Auch in Fällen, in denen ein Intraoralsensor zur Detektion von Biomarkern im Speichel verwendet wird, ist eine Zahnschiene mit Durchgangsöffnungen vorteilhaft, da so für ausreichenden Speichelfluss im Bereich des Sensors gesorgt werden kann, sodass dieser zuverlässige Messergebnisse liefern kann.

Im Zusammenhang mit der vorliegenden Erfindung wird dabei unter einem Biomarker ein Kennwert oder eine Charakteristik verstanden, die objektiv gemessen werden kann und einen biologischen Prozess, einen pathogenen Prozess oder eine pharmakologische Reaktion auf eine therapeutische Intervention beschreibt. Beispiele für Biomarker im Speichel sind ein pH-Wert, eine Salzkonzentration und eine elektrische Leitfähigkeit.

Wie bereits erwähnt, ist ein für sich genommen bekanntes Verfahren zum Herstellen von Zahnschienen das Tiefziehen. Dabei wird eine Folie, aus der die Zahnschiene hergestellt werden soll, unter Einfluss von Druck und Wärme über ein Zahnmodell gezogen, das beispielsweise aus Gips hergestellt ist. Alternativ kann das Zahnmodell mittels eines 3D-Druckverfahrens hergestellt sein. Das Tiefziehverfahren kann jedoch nicht verwendet werden, wenn die tiefzuziehende Folie Löcher oder Durchgangsöffnungen aufweist. Diese Löcher oder Durchgangsöffnungen verhindern, dass der für das Tiefziehen notwendige Überdruck oder Unterdruck aufgebaut werden kann. Zusammengefasst können mit bekannten Tiefziehverfahren keine Zahnschienen hergestellt werden, die Durchgangsöffnungen für Speichel und/oder Nahrungspartikel aufweisen.

Mit dem erfindungsgemäßen Verfahren wurde eine Möglichkeit gefunden, Zahnschienen, welche Durchgangsöffnungen für Speichel und/oder Nahrungspartikel aufweisen, mittels eines Tiefziehverfahrens herzustellen. Somit wurde erreicht, dass sich die oben erwähnten Vorteile von Zahnschienen mit Durchgangsöffnungen mit den Vorteilen des Tiefziehverfahrens kombinieren lassen. Dabei liefern Tiefziehverfahren üblicherweise Zahnschienen mit vergleichsweise hoher Präzision. Die Herstellungszeit für derartige Zahnschienen ist dabei beim Tiefziehen vergleichsweise gering. Dies gilt insbesondere im Vergleich zu Zahnschienen, die durch additive Herstellungsverfahren oder durch spanende Herstellungsverfahren produziert werden. Ferner haben tiefgezogene Zahnschienen den Vorteil, dass beim Tiefziehen üblicherweise keine Grate oder Kanten entstehen, die zu einem verminderten Tragekomfort führen würden. Anders gesagt können mittels Tiefziehverfahren Zahnschienen hergestellt werden, die sich komfortabel tragen lassen.

Einer der Kerngedanken des erfindungsgemäßen Verfahrens besteht dabei darin, mittels der Dichtfolie die Durchgangsöffnungen in der Funktionsfolie derart zu verschließen, dass sich der zum Tiefziehen nötige Überdruck oder Unterdruck mit Wirkung für die Funktionsfolie generieren lässt. Nach dem Tiefziehen wird die Dichtfolie von der Funktionsfolie abgenommen, sodass die Durchgangsöffnungen für Speichel und/oder Nahrungspartikel nicht mehr von der Dichtfolie verschlossen sind und für die oben beschriebenen Funktionen bereitstehen.

Das luftdichte Verschließen der Mehrzahl an Durchgangsöffnungen mittels der Dichtfolie kann ein Bereitstellen der Dichtfolie und der Funktionsfolie als Bestandteile eines mehrschichtigen Folienmaterials umfassen. Alternativ kann das Verschließen der Mehrzahl an Durchgangsöffnungen mittels der Dichtfolie ein Auflegen der Dichtfolie auf die Funktionsfolie umfassen. Das luftdichte Verschließen der Mehrzahl an Durchgangsöffnungen kann also auf verschiedene Arten erfolgen. Gemäß einer ersten Variante wird ein mehrschichtiges Folienmaterial als Ausgangsmaterial für das Tiefziehen bereitgestellt. Dieses mehrschichtige Folienmaterial umfasst die zahnmedizinische Funktionsfolie und die Dichtfolie. Innerhalb des mehrschichtigen Folienmaterials sind die zahnmedizinische Funktionsfolie und die Dichtfolie derart angeordnet und/oder miteinander verbunden, dass die Durchgangsöffnungen der Funktionsfolie mittels der Dichtfolie luftdicht verschlossen sind. Gemäß dieser Variante werden sozusagen die ersten beiden Schritte des oben erwähnten Verfahrens gemeinsam ausgeführt. Nach dem Tiefziehen des mehrschichtigen Folienmaterials wird die Dichtfolie von der Funktionsfolie getrennt, d. h. es wird diejenige Schicht des mehrschichtigen Folienmaterials, die durch die Dichtfolie gebildet wird, von derjenigen Schicht, die durch die Funktionsfolie gebildet wird, getrennt. Gemäß einer zweiten Variante wird die Dichtfolie im Zuge des Verfahrens zum Herstellen der Zahnschiene auf die Funktionsfolie derart aufgelegt, dass die Mehrzahl an Durchgangsöffnungen luftdicht verschlossen ist. Hierzu gibt es zwei Unter-Varianten. Gemäß einer ersten Unter-Variante wird die Dichtfolie als eine separate Folie vorgesehen. Diese wird sozusagen als separates Bauteil auf die Funktionsfolie aufgelegt, um die Durchgangsöffnungen luftdicht zu verschließen. Gemäß einer zweiten Unter-Variante ist die Dichtfolie eine Komponente der Tiefzieh-Anlage oder des Tiefzieh-Geräts. Gemäß dieser Variante ist die Dichtfolie bevorzugt mittels eines Rahmens gehalten, der beweglich gegenüber den übrigen Komponenten der Tiefzieh-Anlage gelagert ist, sodass durch entsprechendes Bewegen des Rahmens die Dichtfolie auf die zahnmedizinische Funktionsfolie aufgelegt werden kann und sodann die Dichtfolie und die zahnmedizinische Funktionsfolie gemeinsam tiefgezogen werden können. Danach wird die Dichtfolie wieder von der zahnmedizinischen Funktionsfolie abgenommen, indem sie beispielsweise mittels des Rahmens von der Funktionsfolie abgehoben wird. Gemäß einer bevorzugten Ausführungsform der zweiten Unter-Variante ist die Dichtfolie mehrfach verwendbar. Sie kann also zum Herstellen mehrerer Zahnschienen, die zeitlich nacheinander hergestellt werden, verwendet werden. In allen genannten Varianten lassen sich die Durchgangsöffnungen der zahnmedizinischen Funktionsfolie zuverlässig luftdicht verschließen, sodass die oben erwähnte Kombination aus den Vorteilen einer Zahnschiene mit Durchgangsöffnungen und den Vorteilen des Tiefziehens einfach und zuverlässig erreicht wird.

Im Zusammenhang mit der vorliegenden Erfindung beträgt eine Dicke oder Stärke der zahnmedizinischen Funktionsfolie beispielsweise 0,5mm bis 5mm. Für den Fall, dass die Funktionsfolie zur Herstellung einer Aligner-Schiene dient, kann die Dicke oder Stärke der Funktionsfolie 0,5mm bis 2mm, bevorzugt 0,5mm bis 1,0mm betragen. In einem Fall, in dem die Funktionsfolie zur Herstellung einer Knirscher- oder Aufbissschiene verwendet wird, kann die Stärke oder Dicke der Funktionsfolie 1,0mm bis 2,5mm betragen. Falls die Funktionsfolie zur Herstellung einer Sportschutzschiene, insbesondere einer Boxerschutzschienen, dient, kann die Stärke oder Dicke 1,5mm bis 5mm betragen.

Bevorzugte Materialien für die zahnmedizinische Funktionsfolie sind Polyethylenterephtalat-Glykol (PET-G), Polyethylenterephtalat (PET), Polyurethane (PU), thermoplastische Polyurethane (TPU), Multilayer PU oder Multilayer TPU. Ebenfalls können Mischungen dieser Materialien verwendet werden. Weitere bevorzugte Materialien sind Polycarbonate, Polymethylmethacrylat (PMMA), Polystyrol (PS), Polypropylen (PP), Polyvinylchlorid (PVC), Polylactid (PLA), Ethylenvinylacetat (EVAC) und Styrol-Butadien-Styrol (SBS). Für die Dichtfolie können dieselben Materialien verwendet werden.

Wie bereits aus den vorstehenden Erläuterungen deutlich wurde, müssen die Durchgangsöffnungen in der zahnmedizinischen Funktionsfolie dazu geeignet sein, einen Austausch von Speichel sowie einen Durchtritt von Nahrungspartikeln zu ermöglichen. Für den Fall, dass die Durchgangsöffnungen einen kreisförmigen Querschnitt haben, beträgt dieser mindestens mehrere Mikrometer. Für den Fall, dass die Durchgangsöffnungen keinen kreisförmigen Querschnitt haben, gilt dies für einen äquivalenten Durchmesser. Dieser wird als Durchmesser eines Kreises errechnet, der denselben Flächeninhalt hat wie ein Querschnitt einer zugehörigen nicht-kreisförmigen Durchgangsöffnung.

Gemäß einer Ausführungsform liegen die Funktionsfolie und die Dichtfolie direkt aneinander an. Alternativ sind die Funktionsfolie und die Dichtfolie mittels einer Verbindungsschicht verbunden. Die erste Alternative gilt sowohl für diejenige Variante, in der die Funktionsfolie und die Dichtfolie als Bestandteile eines mehrschichtigen Folienaufbaus vorgesehen werden als auch für diejenige Variante, in der die Dichtfolie als separate Komponente bereitgestellt wird. Die zweite Alternative ist insbesondere mit derjenigen Variante kombiniert, in der die Funktionsfolie und die Dichtfolie als Bestandteile eines mehrschichtigen Folienaufbaus vorgesehen werden. In beiden Varianten wird erreicht, dass mittels der Dichtfolie die Durchgangsöffnungen in der Funktionsfolie zuverlässig luftdicht verschlossen werden. Gemäß einer Variante ist die Dichtfolie oder die Verbindungsschicht wasserlöslich. Das Abnehmen der Dichtfolie von der Funktionsfolie umfasst ein Auflösen der wasserlöslichen Dichtfolie oder der wasserlöslichen Verbindungsschicht. In einer Ausführungsform, in der die Funktionsfolie und die Dichtfolie mittels einer Verbindungsschicht verbunden sind, kann die Verbindungsschicht wasserlöslich sein. In diesem Fall kann die Dichtfolie von der Funktionsfolie abgenommen werden, indem die Verbindungsschicht mittels Wasser aufgelöst wird. Auf diese Weise lässt sich besonders zuverlässig ein luftdichtes Verschließen der Durchgangsöffnungen mit einer einfachen Abnehmbarkeit der Dichtfolie kombinieren. In einer anderen Ausführungsform, in der die Dichtfolie direkt an der Funktionsfolie anliegt, kann die Dichtfolie wasserlöslich sein. In diesem Fall kann die Dichtfolie von der Funktionsfolie abgenommen werden, indem die Dichtfolie mittels Wasser aufgelöst wird. Auch auf diese Weise lässt sich besonders zuverlässig ein luftdichtes Verschließen der Durchgangsöffnungen mit einer einfachen Abnehmbarkeit der Dichtfolie kombinieren.

Eine wasserlösliche Dichtfolie oder eine wasserlösliche Verbindungsschicht umfasst beispielsweise Polymere wie Polyvinylalkohol (PVOH bzw. PVA), Butendiol-Vinylalkohol-Copolymer (BVOH), Cellulose, Hypromellose, Ethylcellulose, oder einen wasserlöslichen Wirkstoff.

In einem Beispiel umfasst das Verfahren ferner ein Herstellen einer Sensorschnittstelle in der Funktionsfolie, wobei wenigstens ein Teil der Mehrzahl an Durchgangsöffnungen im Bereich der Sensorschnittstelle angeordnet ist. In diesem Zusammenhang wird unter einer Sensorschnittstelle ein Abschnitt der Zahnschiene verstanden, der dazu vorgesehen ist, an einem Sensor anzuliegen oder anderweitig mit einem Sensor gekoppelt zu werden. Bei dem Sensor handelt es sich um einen Intraoralsensor, der der Detektion von Biomarkern im Speichel dient. Die Sensorschnittstelle kann beispielsweise als Vertiefung oder Aufnahmetasche ausgebildet sein. Die Vertiefung ist dabei insbesondere für Sensoren gut geeignet, die direkt auf einem Zahn platziert werden. In einem Fall, in dem der Sensor in einer Zahnkrone oder in einer Zahnbrücke verbaut ist, kann die Sensorschnittstelle lediglich als ein Abschnitt der Zahnschiene ausgebildet sein, der am Sensor anliegt. In allen Varianten muss durch die Durchgangsöffnungen sichergestellt sein, dass ausreichend Speichel zum Sensor fließt. Hierzu kann die Sensorschnittstelle eine Gruppe oder ein Cluster von Durchgangsöffnungen aufweisen. Alternativ oder zusätzlich können sich die Durchgangsöffnungen der Sensorschnittstelle von den übrigen Durchgangsöffnungen unterscheiden, z. B. hinsichtlich Form und/oder Größe.

Wenigstens ein Teil der Mehrzahl an Durchgangsöffnungen in der Funktionsfolie kann patientenindividuell positioniert und/oder gestaltet sein. Insbesondere sind dabei alle Durchgangsöffnungen patientenindividuell positioniert und/oder gestaltet. Die patientenindividuelle Gestalt schließt dabei insbesondere patientenindividuelle Querschnittsformen und/oder Querschnittsgrößen der Durchgangsöffnungen ein. Dabei meint patientenindividuell, dass die Durchgangsöffnungen gemäß Merkmalen angeordnet und/oder gestaltet sind, die denjenigen Patienten charakterisieren, für den die Zahnschiene hergestellt wird. Diese Merkmale sind spezifisch für diesen Patienten, d. h. bei einem anderen Patienten sind diese Merkmale anders ausgeprägt. Auf diese Weise kann beispielsweise berücksichtigt werden, dass ein Patient künstliche Zähne hat, Zahnlücken hat o. ä. Durch die patientenindividuelle Anordnung und/oder Gestalt der Durchgangsöffnungen wird erreicht, dass der Speichelaustausch sowie die Durchlässigkeit für Nahrungspartikel für den gegebenen Patienten besonders gut funktioniert.

Wenigstens ein Teil der Mehrzahl an Durchgangsöffnungen in der Funktionsfolie kann basierend auf wenigstens einem Anatomiemerkmal positioniert und/oder gestaltet sein. Insbesondere sind alle Durchgangsöffnungen basierend auf wenigstens einem Anatomiemerkmal positioniert und/oder gestaltet. In diesem Zusammenhang können beispielsweise im Bereich der Spitzen von Zahnhöckern Durchgangsöffnungen vorgesehen sein, um den Speichelabfluss in diesem Bereich sicherzustellen. Ebenso ist es möglich, Durchgangsöffnungen im Bereich der Zahnzwischenräume, im flächigen Okklusalbereich, am Übergang zwischen Zähnen und Zahnfleisch und/oder im Bereich der Zahnflanken vorzusehen. Die Durchgangsöffnungen in einer Zahnschiene für einen Oberkiefer können sich dabei hinsichtlich Positionierung und/oder Gestalt von Durchgangsöffnungen in einer Zahnschiene für einen Unterkiefer unterscheiden. Insgesamt können die Durchgangsöffnungen also hinsichtlich Position und Gestalt auf die zahnmedizinische Anatomie abgestimmt werden, sodass unerwünschte Einflüsse der Zahnschiene minimiert und ein Tragekomfort maximiert wird.

Wenigstens ein Teil der Mehrzahl an Durchgangsöffnungen in der Funktionsfolie kann eine Sollbruchstelle zum Trennen der Zahnschiene von überschüssiger Funktionsfolie bilden. Mittels einer derartigen Sollbruchstelle können also nach dem Tiefziehen diejenigen Abschnitte der Funktionsfolie, die die Zahnschiene bilden, von den übrigen Abschnitten der Funktionsfolie getrennt werden. Dasselbe gilt für die Dichtfolie. Dieser Trennvorgang ist aufgrund der Sollbruchstelle besonders einfach und zuverlässig. Darüber hinaus kann die Sollbruchstelle patientenindividuell angeordnet und/oder gestaltet werden. Somit ergibt sich ohne weitere Nachbearbeitungsschritte ein hoher Tragekomfort der Zahnschiene.

Es versteht sich, dass insbesondere in Fällen, in denen wenigstens ein Teil der Mehrzahl an Durchgangsöffnungen im Bereich der Sensorschnittstelle angeordnet ist, in denen wenigstens ein Teil der Mehrzahl an Durchgangsöffnungen in der Funktionsfolie patientenindividuell positioniert und/oder gestaltet ist, in denen wenigstens ein Teil der Mehrzahl an Durchgangsöffnungen in der Funktionsfolie basierend auf wenigstens einem Anatomiemerkmal positioniert und/oder gestaltet ist und/oder in denen wenigstens ein Teil der Mehrzahl an Durchgangsöffnungen in der Funktionsfolie eine Sollbruchstelle zum Trennen der Zahnschiene von überschüssiger Funktionsfolie bildet, die Positionierung und Orientierung der Funktionsfolie auf dem Zahnmodell beim Tiefziehen entlang des Zahnmodells entscheidend dafür ist, dass die Durchgangsöffnungen nach dem Tiefziehen an der gewünschten Position sitzen. Nur so können die Durchgangsöffnungen die gewünschte Funktion in gewünschter Art und Weise erfüllen. Zu diesem Zweck kann auf der Funktionsfolie und/oder einer mit dieser verbundenen Folie, d.h. der Dichtfolie, der Verbindungsschicht und/oder der Schutzfolie, eine Positionierhilfe vorgesehen sein. Außerdem kann zu diesem Zweck auf dem Zahnmodell, d.h. auf dem Tiefziehwerkzeug, eine Positionierhilfe vorgesehen sein. Die Positionierhilfe auf der Funktionsfolie und/oder einer mit dieser verbundenen Folie und die Positionierhilfe auf dem Zahnmodell können dazu ausgebildet sein, zum Positionieren und/oder Orientieren der Funktionsfolie auf dem Zahnmodell zusammenzuwirken. Dabei können beide Positionierhilfen beispielsweise als optische Markierung oder Muster ausgeführt sein. Es lässt sich somit die Funktionsfolie präzise und korrekt auf dem Zahnmodell positionieren und/oder orientieren, sodass sich die Durchgangsöffnungen innerhalb der herzustellenden Zahnschiene präzise anordnen lassen.

Gemäß eines Beispiels umfasst dabei das erfindungsgemäße Verfahren das Einbringen oder Aufbringen einer Positionierhilfe auf die Funktionsfolie und/oder eine mit dieser verbundenen Folie.

Wenigstens ein Teil der Mehrzahl an Durchgangsöffnungen in der Funktionsfolie kann basierend auf einer in der tiefgezogenen Zahnschiene festgelegten Position, Größe und/oder Form in der Funktionsfolie derart verzerrt sein, dass sich nach dem Tiefziehen die festgelegte Position, Größe und/oder Form ergibt. Es versteht sich, dass die Größe, Position und/oder Form der Durchgangsöffnungen in der fertigen Zahnschiene relevant sind. Das gilt insbesondere, wenn die Durchgangsöffnungen patientenindividuell positioniert und/oder gestaltet und/oder basierend auf wenigstens einem Anatomiemerkmal positioniert und/oder gestaltet sind. Dasselbe gilt für die optionale Sollbruchstelle. Die Positionierung und Gestaltung der Durchgangsöffnungen in der Funktionsfolie erfolgt daher sozusagen mittels einer Umrechnung. Das bedeutet, dass ermittelt wird, welche Position und/oder Gestalt die Durchgangsöffnungen in der Funktionsfolie haben müssen, sodass sich nach dem Tiefziehen die gewünschte Position und/oder Gestalt ergibt. Hierfür kann eine Tiefziehsimulation durchgeführt werden. Mittels einer derartigen Simulation können die Durchgangsöffnungen sozusagen aus der Zweidimensionalität der Funktionsfolie in die Dreidimensionalität der Zahnschiene umgerechnet werden.

Das Bereitstellen der zahnmedizinischen Funktionsfolie kann ferner umfassen:
- Bereitstellen der Funktionsfolie in einem unperforierten Zustand, und
- Einbringen der Mehrzahl an Durchgangsöffnungen in die Funktionsfolie.

Gemäß dieser Ausführungsform umfasst das Verfahren zum Herstellen der Zahnschiene also auch das Einbringen der Durchgangsöffnungen in die Funktionsfolie. Hierzu wird ein unperforierter Funktionsfolienrohling bereitgestellt, also eine Folie, die bis auf die Durchgangsöffnungen der Funktionsfolie entspricht. Die Durchgangsöffnungen können dann mittels Stanzen, Lasern, Fräsen, Bohren, Ätzen oder anderen geeigneten Verfahren in den Funktionsfolienrohling eingebracht werden, um diesen zur Funktionsfolie zu machen. Diese Schritte werden vor dem Bereitstellen des Folienmaterials oder im Zuge des Bereitstellens des Folienmaterials ausgeführt.

Gemäß einer Variante kann eine Schutzfolie vorgesehen sein, die während des Tiefziehens zwischen der zahnmedizinischen Funktionsfolie und dem Zahnmodell angeordnet ist, um eine negative Beeinflussung, z. B. Verschmutzung oder Beschädigung, der Funktionsfolie durch das Zahnmodell zu verhindern. Die Schutzfolie kann als Bestandteil des mehrschichtigen Folienmaterials vorgesehen sein oder kann als separate Komponente vor dem Tiefziehen zwischen das Zahnmodell und die Funktionsfolie gelegt werden.

Für die Schutzfolie können dieselben Materialien verwendet werden, die weiter oben bereits für die Funktionsfolie und die Dichtfolie erwähnt wurden.

In einem Beispiel umfasst das mehrschichtige Folienmaterial also eine Schutzfolie, eine zahnmedizinische Funktionsfolie und eine Dichtfolie, wobei die Schutzfolie und die Dichtfolie auf unterschiedlichen Seiten auf die Funktionsfolie aufgebracht sind. Optional kann die Schutzfolie und/oder die Dichtfolie mittels einer Verbindungsschicht mit der Funktionsfolie verbunden sein.

Optional kann eine kombinierte Schutzfolie und Dichtfolie vorgesehen sein, die auch als Kombinationsfolie bezeichnet werden kann. Das bedeutet, dass eine einzige Folie die Funktion der Schutzfolie und der Dichtfolie erfüllt. Diese Kombinationsfolie muss somit beim Tiefziehen zwischen der zahnmedizinischen Funktionsfolie und dem Zahnmodell angeordnet sein. Wie zuvor erläutert, kann dabei die Kombinationsfolie ein Bestandteil eines mehrschichtigen Folienmaterials sein. Alternativ kann die Kombinationsfolie als separate Komponente vorgesehen werden. Aufgrund der Positionierung der Kombinationsfolie zwischen dem Zahnmodell und der Funktionsfolie muss die Kombinationsfolie möglichst dünn sein, da ansonsten die Passgenauigkeit der aus der Funktionsfolie hergestellten Zahnschiene beeinträchtigt werden kann.

Zudem wird die Aufgabe durch eine Zahnschiene gelöst, die mittels des erfindungsgemäßen Verfahrens erhalten ist. Wie bereits erwähnt, sind Zahnschienen mit Durchgangsöffnungen vorteilhaft. Auf die vorstehenden Erläuterungen dieser Vorteile wird verwiesen. Darüber hinaus ist eine Herstellung einer Zahnschiene mittels eines Tiefziehverfahrens vorteilhaft, wie bereits oben erläutert wurde. Auf diese Erläuterungen wird ebenfalls verwiesen. Die mittels des erfindungsgemäßen Verfahrens erhaltene Zahnschiene vereint somit in sich die Vorteile, die aus den Durchgangsöffnungen resultieren, und die Vorteile, die aus einer Herstellung mittels eines Tiefziehverfahrens resultieren. Neben den bereits erwähnten Vorteilen tiefgezogener Zahnschienen ist die mechanische Flexibilität sowie die vergleichsweise gleichbleibende Schichtstärke bei tiefgezogenen Schienen wichtig. Beides trägt einem hohen Tragekomfort und einer leichten Handhabung bei. Diese Vorteile bestehen insbesondere gegenüber spanend hergestellten Zahnschienen oder additiv hergestellten Zahnschienen, die üblicherweise eine geringere mechanische Flexibilität haben. Ferner können spanend hergestellte Zahnschienen oder additiv hergestellten Zahnschienen Kanten, Absätze und/oder Sprünge aufweisen, die dem Tragekomfort abträglich sind. Neben diesen Kriterien kann eine tiefgezogene Zahnschiene zudem anhand ihrer mikroskopischen Struktur von spanend oder additiv hergestellten Zahnschienen unterschieden werden. Das gilt einerseits für das Gefüge und andererseits hinsichtlich typischer Zerspanungsspuren, z. B. Frässpuren, in spanend hergestellten Zahnschienen und Slices oder Schichten in additiv gefertigten Zahnschienen. Ferner kann man in spanend hergestellten Zahnschienen häufig einen Werkzeugradius als Mindestradius von Unterschnitten erkennen. Auch anhand der verwendeten Materialien können tiefgezogene Zahnschienen von spanend gefertigten oder additiv gefertigten Zahnschiene unterschieden werden. Die tiefgezogenen Zahnschienen nutzen in der Regel thermoplastische Kunststoffe. Spanend hergestellte Zahnschienen umfassen typischerweise (Meth)acrylate oder Epoxidharze. Dabei muss insbesondere berücksichtigt werden, dass mittels SLS-Druck hergestellte Zahnschienen Photoinitiatoren enthalten, was bei Folien für Tiefziehschienen nicht der Fall ist. Somit lassen sich anhand einer Detektion der Photoinitiatoren mittels SLS-Druck hergestellte Zahnschienen von tiefgezogenen Zahnschienen unterscheiden.

Außerdem wird die Aufgabe durch eine Zahnschienenbaugruppe gelöst, welche einen Zahnschienenkörper umfasst, der aus einer zahnmedizinischen Funktionsfolie gebildet ist, die eine Mehrzahl an Durchgangsöffnungen für Speichel und/oder Nahrungspartikel aufweist. Dabei sind die Durchgangsöffnungen mittels einer Dichtfolie luftdicht verschlossen. Mit anderen Worten betrifft die Zahnschienenbaugruppe dasjenige Zwischenprodukt beim Herstellen der Zahnschiene mittels des erfindungsgemäßen Verfahrens, bei dem die Funktionsfolie und die Dichtfolie bereits tiefgezogen sind, die Dichtfolie jedoch noch nicht von der zahnmedizinischen Funktionsfolie abgenommen ist. Auch in der Zahnschienenbaugruppe werden die bereits erläuterten Vorteile einer Zahnschiene mit Durchgangsöffnungen und die Vorteile eines Tiefziehverfahrens kombiniert.

Auch wird die Aufgabe durch ein Folienmaterial zum Herstellen einer Zahnschiene gelöst. Das Folienmaterial umfasst eine zahnmedizinische Funktionsfolie, in der eine Mehrzahl an Durchgangsöffnungen für Speichel und/oder Nahrungspartikel vorgesehen ist, und eine Dichtfolie, welche die Mehrzahl an Durchgangsöffnungen luftdicht verschließt. Die zahnmedizinische Funktionsfolie und die Dichtfolie werden also als mehrschichtiges Folienmaterial bereitgestellt. Aufgrund der Anwendung zur Herstellung von Zahnschienen kann dieses Folienmaterial ferner als ein dentales Folienmaterial bezeichnet werden. Dieses Folienmaterial kann in vergleichsweise einfacher und effizienter Weise als Ausgangsmaterial für das erfindungsgemäße Verfahren zum Herstellen einer Zahnschiene verwendet werden. Nachdem die zahnmedizinische Funktionsfolie und die Dichtfolie bereits als Bestandteile des mehrschichtigen Folienmaterials bereitgestellt werden, ist es im Zuge des Verfahrens zum Herstellen der Zahnschiene nicht mehr nötig, die Dichtfolie separat handzuhaben. Dadurch wird das Verfahren einfach und effizient. Die Verwendung dieses mehrschichtigen Folienmaterials als Ausgangsmaterial ermöglicht somit, eine Zahnschiene mit Durchgangsöffnungen herzustellen und dabei die Vorteile eines Tiefziehverfahrens zu nutzen.

Im Folienmaterial können die Funktionsfolie und die Dichtfolie direkt aneinander anliegen. Alternativ können die Funktionsfolie und die Dichtfolie mittels einer Verbindungsschicht verbunden sein. In beiden Varianten wird erreicht, dass mittels der Dichtfolie die Durchgangsöffnungen in der Funktionsfolie zuverlässig luftdicht verschlossen werden. Ferner ist in beiden Varianten das Folienmaterial ein einfach handzuhabendes Ausgangsmaterial zum Herstellen einer Zahnschiene mit Durchgangsöffnungen.

Gemäß einer Variante ist die Dichtfolie oder die Verbindungsschicht wasserlöslich. In einer Ausführungsform, in der die Funktionsfolie und die Dichtfolie mittels einer Verbindungsschicht verbunden sind, kann die Verbindungsschicht wasserlöslich sein. In diesem Fall kann die Dichtfolie von der Funktionsfolie abgenommen werden, indem die Verbindungsschicht mittels Wasser aufgelöst wird. Auf diese Weise lässt sich besonders zuverlässig ein luftdichtes Verschließen der Durchgangsöffnungen mit einer einfachen Abnehmbarkeit der Dichtfolie kombinieren. In einer anderen Ausführungsform, in der die Dichtfolie direkt an der Funktionsfolie anliegt, kann die Dichtfolie wasserlöslich sein. In diesem Fall kann die Dichtfolie von der Funktionsfolie abgenommen werden, indem die Dichtfolie mittels Wasser aufgelöst wird. Auch auf diese Weise lässt sich besonders zuverlässig ein luftdichtes Verschließen der Durchgangsöffnungen mit einer einfachen Abnehmbarkeit der Dichtfolie kombinieren.

Wenigstens ein Teil der Mehrzahl an Durchgangsöffnungen kann in einem Bereich einer Sensorschnittstelle für einen Sensor zur Detektion eines Biomarkers im Speichel angeordnet sein. Alternativ oder zusätzlich kann wenigstens ein Teil der Mehrzahl an Durchgangsöffnungen patientenindividuell positioniert und/oder gestaltet sein. Weiter alternativ oder zusätzlich kann wenigstens ein Teil der Mehrzahl an Durchgangsöffnungen basierend auf wenigstens einem Anatomiemerkmal positioniert und/oder gestaltet sein. In diesem Zusammenhang wird unter einer Sensorschnittstelle wieder ein Abschnitt der Zahnschiene verstanden, der dazu vorgesehen ist, an einem Sensor anzuliegen oder anderweitig mit einem Sensor gekoppelt zu werden. Bei dem Sensor handelt es sich um einen Intraoralsensor, der der Detektion von Biomarkern im Speichel dient. Die Sensorschnittstelle kann beispielsweise als Vertiefung oder Aufnahmetasche ausgebildet sein. Die Vertiefung ist dabei insbesondere für Sensoren gut geeignet, die direkt auf einem Zahn platziert werden. In einem Fall, in dem der Sensor in einer Zahnkrone oder in einer Zahnbrücke verbaut ist, kann die Sensorschnittstelle lediglich als ein Abschnitt der Zahnschiene ausgebildet sein, der am Sensor anliegt. In allen Varianten muss durch die Durchgangsöffnungen sichergestellt sein, dass ausreichend Speichel zum Sensor fließt. Hierzu kann die Sensorschnittstelle eine Gruppe oder ein Cluster von Durchgangsöffnungen aufweisen. Alternativ oder zusätzlich können sich die Durchgangsöffnungen der Sensorschnittstelle von den übrigen Durchgangsöffnungen unterscheiden. Indem dies bereits im Folienmaterial berücksichtigt ist, das als Ausgangsmaterial zur Herstellung der Zahnschiene verwendet wird, wird eine Herstellung der Zahnschiene erleichtert. Alternativ oder zusätzlich kann wenigstens ein Teil der Mehrzahl an Durchgangsöffnungen patientenindividuell positioniert und/oder gestaltet sein. Insbesondere sind dabei alle Durchgangsöffnungen patientenindividuell positioniert und/oder gestaltet. Die patientenindividuelle Gestalt schließt dabei insbesondere eine patientenindividuelle Querschnittsform der Durchgangsöffnungen ein. Dabei meint patientenindividuell, dass die Durchgangsöffnungen gemäß Merkmalen angeordnet und/oder gestaltet sind, die denjenigen Patienten charakterisieren, für den die Zahnschiene hergestellt wird. Diese Merkmale sind spezifisch für diesen Patienten, d. h. bei einem anderen Patienten sind diese Merkmale anders ausgeprägt. Auf diese Weise kann beispielsweise berücksichtigt werden, dass ein Patient künstliche Zähne hat, Zahnlücken hat o. ä. Durch die patientenindividuelle Anordnung und/oder Gestalt der Durchgangsöffnungen wird erreicht, dass der Speichelaustausch sowie die Durchlässigkeit für Nahrungspartikel für den gegebenen Patienten besonders gut funktioniert. Indem dies bereits im Folienmaterial berücksichtigt ist, das als Ausgangsmaterial zur Herstellung der Zahnschiene verwendet wird, wird eine Herstellung der Zahnschiene erleichtert. Alternativ oder zusätzlich kann wenigstens ein Teil der Mehrzahl an Durchgangsöffnungen basierend auf wenigstens einem Anatomiemerkmal positioniert und/oder gestaltet sein. Insbesondere sind alle Durchgangsöffnungen basierend auf wenigstens einem Anatomiemerkmal positioniert und/oder gestaltet. In diesem Zusammenhang können beispielsweise im Bereich der Spitzen von Zahnhöckern Durchgangsöffnungen vorgesehen sein, um den Speichelabfluss in diesem Bereich sicherzustellen. Ebenso ist es möglich, Durchgangsöffnungen im Bereich der Zahnzwischenräume, im flächigen Okklusalbereich, am Übergang zwischen Zähnen und Zahnfleisch und/oder im Bereich der Zahnflanken vorzusehen. Die Durchgangsöffnungen in einer Zahnschiene für einen Oberkiefer können sich dabei hinsichtlich Positionierung und/oder Gestalt von Durchgangsöffnungen in einer Zahnschiene für einen Unterkiefer unterscheiden. Insgesamt können die Durchgangsöffnungen also hinsichtlich Position und/oder Gestalt auf die zahnmedizinische Anatomie abgestimmt werden, sodass unerwünschte Einflüsse der Zahnschiene minimiert und ein Tragekomfort maximiert wird. Indem dies bereits im Folienmaterial berücksichtigt ist, das als Ausgangsmaterial zur Herstellung der Zahnschiene verwendet wird, wird eine Herstellung der Zahnschiene erleichtert.

Gemäß einer weiteren Ausführungsform des Folienmaterials kann wenigstens ein Teil der Mehrzahl an Durchgangsöffnungen in der Funktionsfolie eine Sollbruchstelle zum Trennen der Zahnschiene von überschüssiger Funktionsfolie bilden. Mittels einer derartigen Sollbruchstelle können also nach dem Tiefziehen diejenigen Abschnitte der Funktionsfolie, die die Zahnschiene bilden, von den übrigen Abschnitten der Funktionsfolie getrennt werden. Dasselbe gilt für die Dichtfolie. Dieser Trennvorgang ist aufgrund der Sollbruchstelle besonders einfach und zuverlässig. Darüber hinaus kann die Sollbruchstelle patientenindividuell angeordnet und/oder gestaltet werden. Somit ergibt sich ohne weitere Nachbearbeitungsschritte ein hoher Tragekomfort der Zahnschiene.

Wenigstens ein Teil der Mehrzahl an Durchgangsöffnungen in der Funktionsfolie des Folienmaterials kann basierend auf einer in der tiefgezogenen Zahnschiene festgelegten Position, Größe und/oder Form in der Funktionsfolie derart verzerrt sein, dass sich nach dem Tiefziehen die festgelegte Position, Größe und/oder Form ergibt. Es versteht sich, dass die Größe, Position und/oder Form der Durchgangsöffnungen in der fertigen Zahnschiene relevant sind. Das gilt insbesondere, wenn die Durchgangsöffnungen patientenindividuell positioniert und/oder gestaltet und/oder basierend auf wenigstens einem Anatomiemerkmal positioniert und/oder gestaltet sind. Dasselbe gilt für die optionale Sollbruchstelle. Die Positionierung und Gestaltung der Durchgangsöffnungen in der Funktionsfolie erfolgt daher sozusagen mittels einer Umrechnung. Das bedeutet, dass ermittelt wird, welche Position und/oder Gestalt die Durchgangsöffnungen in der Funktionsfolie haben müssen, sodass sich nach dem Tiefziehen die gewünschte Position und/oder Gestalt ergibt. Hierfür kann eine Tiefziehsimulation durchgeführt werden. Mittels einer derartigen Simulation können die Durchgangsöffnungen sozusagen aus der Zweidimensionalität der Funktionsfolie in die Dreidimensionalität der Zahnschiene umgerechnet werden.

Die Erfindung wird nachstehend anhand verschiedener Ausführungsbeispiele erläutert, die in den beigefügten Zeichnungen gezeigt sind. Es zeigen:
- Figur 1: eine erfindungsgemäße Zahnschiene, die mittels eines erfindungsgemäßen Verfahrens aus einem erfindungsgemäßen Folienmaterial hergestellt ist, wobei die Zahnschiene als Aligner-Schiene ausgebildet ist,
- Figur 2: eine weitere erfindungsgemäße Zahnschiene, die mittels eines erfindungsgemäßen Verfahrens aus einem erfindungsgemäßen Folienmaterial hergestellt ist, wobei die Zahnschiene als Trägerschiene für einen Intraoralsensor zur Detektion eines Biomarkers im Speichel ausgebildet ist, mit Intraoralsensor,
- Figur 3: die Zahnschiene mit Intraoralsensor aus Figur 2 aus einer anderen Blickrichtung,
- Figur 4: ein erfindungsgemäßes Folienmaterial gemäß einem ersten Beispiel in einer vergrößerten Querschnittsdarstellung,
- Figur 5: ein erfindungsgemäßes Folienmaterial gemäß einem zweiten Beispiel in einer vergrößerten Querschnittsdarstellung,
- Figuren 6 bis 8: Illustrationen von Schritten des erfindungsgemäßen Verfahrens zum Herstellen einer Zahnschiene, und
- Figur 9: eine Illustration eines erfindungsgemäßen Verfahrens zum Herstellen einer Zahnschiene gemäß einer Variante.

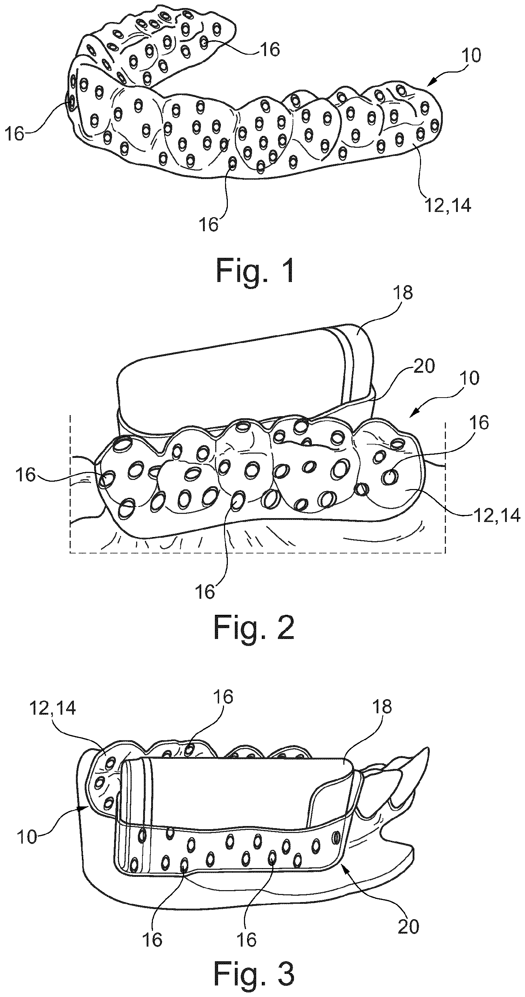

Figur 1 zeigt eine Zahnschiene 10, die als Aligner-Schiene ausgeführt ist.

Die Zahnschiene 10 weist einen Zahnschienenkörper 12 auf, der aus einer zahnmedizinischen Funktionsfolie 14 durch Tiefziehen über ein Zahnmodell gebildet ist. Im vorliegenden Fall wurde das Zahnmodell mittels eines 3D-Druckverfahrens hergestellt.

Die Funktionsfolie 14 und somit der Zahnschienenkörper 12 umfasst eine Mehrzahl an Durchgangsöffnungen 16, von denen der besseren Übersichtlichkeit wegen nur manche mit einem Bezugszeichen versehen sind.

Dabei sind die Durchgangsöffnungen 16 dazu ausgebildet, einen Speichelfluss von einer Seite des Zahnschienenkörpers 12 auf eine andere Seite des Zahnschienenkörpers 12 zu ermöglichen, d. h. von einer den Zähnen zugewandten Seite auf eine den Zähnen abgewandten Seite und umgekehrt. Darüber hinaus ermöglichen die Durchgangsöffnungen 16, dass Nahrungspartikel hindurchtreten.

Im dargestellten Ausführungsbeispiel haben die Durchgangsöffnungen 16 einen Durchmesser von mehreren Mikrometern bis wenigen Millimetern.

In den Figuren 2 und 3 ist ein weiteres Beispiel der Zahnschiene 10 gezeigt. Dabei dient im Beispiel der Figuren 2 und 3 die Zahnschiene 10 als Trägerschiene für einen Intraoralsensor 18 zur Detektion eines Biomarkers. Dabei ist auch der Intraoralsensor 18 dargestellt, der die Form einer Box hat.

Der Aufbau der Zahnschiene 10 aus den Figuren 2 und 3 entspricht im Wesentlichen dem Aufbau der Zahnschiene 10 aus der Figur 1, d. h. auch die Zahnschiene 10 aus den Figuren 2 und 3 weist einen Zahnschienenkörper 12 auf, der aus einer zahnmedizinischen Funktionsfolie 14 durch Tiefziehen über ein Zahnmodell gebildet ist.

Dabei haben die Funktionsfolie 14 und somit der Zahnschienenkörper 12 wieder eine Mehrzahl an Durchgangsöffnungen 16 für Speichel und Nahrungspartikel.

Im Unterschied zur Zahnschiene 10 gemäß Figur 1 erstreckt sich die Zahnschiene 10 aus den Figuren 2 und 3 jedoch nur über einige wenige Zähne, nicht über alle.

Ein weiterer Unterschied besteht darin, dass an der Zahnschiene 10 aus den Figuren 2 und 3 eine Aufnahmetasche 20 für den Intraoralsensor 18 ausgebildet ist. Die Aufnahmetasche 20 ist ebenfalls aus zahnmedizinischer Funktionsfolie 14 mittels eines Tiefziehverfahrens hergestellt. Dabei wird die Aufnahmetasche 20 im Zuge desselben Tiefziehverfahrens geschaffen, in dem auch die Zahnschiene 10 produziert wird.

Das Verfahren zum Herstellen der Zahnschienen 10 aus den Figuren 1 bis 3 wird im Folgenden erläutert. Die Zahnschienen 10 aus den Figuren 1-3 sind also mittels eines solchen Verfahrens erhalten.

Als Ausgangsmaterial für das Verfahren dient ein Folienmaterial 22.

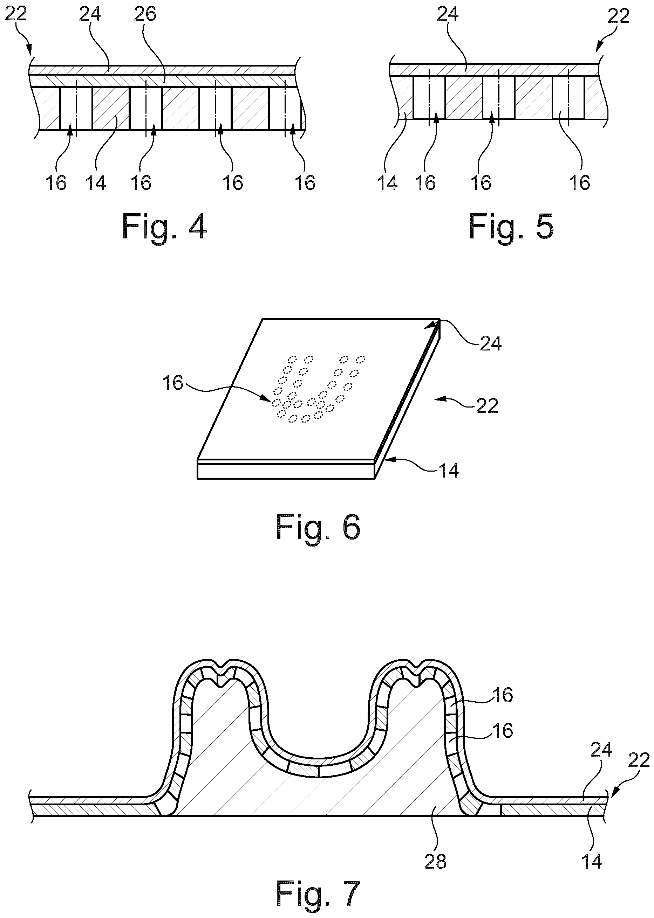

Eine erste Variante des Folienmaterials 22 ist in Figur 4 dargestellt.

In dieser Variante umfasst das Folienmaterial 22 die zahnmedizinische Funktionsfolie 14, in der eine Mehrzahl an Durchgangsöffnungen 16 für Speichel und/oder Nahrungspartikel vorgesehen sind.

Darüber hinaus umfasst das Folienmaterial 22 eine Dichtfolie 24 und eine Verbindungsschicht 26.

Dabei ist die Verbindungsschicht 26 zwischen der Funktionsfolie 14 der Dichtfolie 24 angeordnet, sodass die Funktionsfolie 14 und die Dichtfolie 24 mittels der Verbindungsschicht 26 verbunden sind.

Ferner verschließt die Dichtfolie 24 die Durchgangsöffnungen 16 in der Funktionsfolie 14 luftdicht auf einer Seite.

Dabei kann die Verbindungsschicht 26 wasserlöslich sein, sodass durch Auflösen der Verbindungsschicht 26 die Dichtfolie 24 von der Funktionsfolie 14 abgenommen werden kann, wie später noch im Detail erläutert werden wird.

Figur 5 zeigt eine zweite Variante des Folienmaterials 22. Diese unterscheidet sich dahingehend von der Variante aus Figur 4, dass die Funktionsfolie 14 und die Dichtfolie 24 direkt aneinander anliegen.

In der zweiten Variante kann die Dichtfolie 24 wasserlöslich sein, sodass die Dichtfolie 24 durch Auflösen mittels Wasser von der Funktionsfolie 14 abgenommen werden kann, wie später noch im Detail erläutert werden wird.

In einem ersten Schritt des Verfahrens zum Herstellen der Zahnschiene 10 wird somit das Folienmaterial 22, das gemäß Figur 4 oder gemäß Figur 5 ausgebildet sein kann, bereitgestellt.

Genauer gesagt wird die zahnmedizinische Funktionsfolie 14, aus der später die Zahnschiene 10 entstehen soll, bereitgestellt, wobei in der zahnmedizinischen Funktionsfolie 14 eine Mehrzahl an Durchgangsöffnungen 16 für Speichel und/oder Nahrungspartikel vorhanden ist.

Der besseren Übersichtlichkeit wegen sind dabei in der Figur 6, in der dieser erste Schritt illustriert ist, nur einige Durchgangsöffnungen 16 mit einem Bezugszeichen versehen.

Dabei sind die Durchgangsöffnungen 16 mittels der Dichtfolie 24 luftdicht verschlossen.

In einem nachfolgenden zweiten Schritt werden die Funktionsfolie 14 und die Dichtfolie 24 gemeinsam entlang eines Zahnmodells 28 tiefgezogen.

Dieser Schritt ist in Figur 7 illustriert, wobei das Zahnmodell 28, die Funktionsfolie 14 und die Dichtfolie 24 geschnitten dargestellt sind. Es versteht sich dabei, dass in diesem Zusammenhang wieder sowohl das Folienmaterial 22 aus der Figur 4 als auch das Folienmaterial 22 aus der Figur 5 verwendet werden kann, obwohl in der Figur 7 keine Verbindungsschicht 26 dargestellt ist.

Nachdem das Tiefziehen von Folienmaterialien für sich genommen bekannt ist, sind die Komponenten einer Anlage zum Tiefziehen eines Folienmaterials 22 nicht dargestellt.

Bedeutsam ist allerdings, dass sich das Folienmaterial 22 nur deshalb mittels Tiefziehens verarbeiten lässt, weil die Durchgangsöffnungen 16 mittels der Dichtfolie 24 luftdicht verschlossen sind. Andernfalls wäre es nicht möglich, den für das Tiefziehen nötigen Überdruck oder Unterdruck auf das Folienmaterial 22 und insbesondere auf die zahnmedizinische Funktionsfolie 14 aufzubringen.

Nach dem Tiefziehen liegt also der Zahnschienenkörper 12 vor, der aus der zahnmedizinischen Funktionsfolie 14 gebildet ist und eine Mehrzahl an Durchgangsöffnungen 16 aufweist. Dabei sind die Durchgangsöffnungen 16 mittels der Dichtfolie 24 luftdicht verschlossen.

Diese Kombination aus Zahnschienenkörper 12 und Dichtfolie 24 kann auch als Zahnschienenbaugruppe 30 bezeichnet werden.

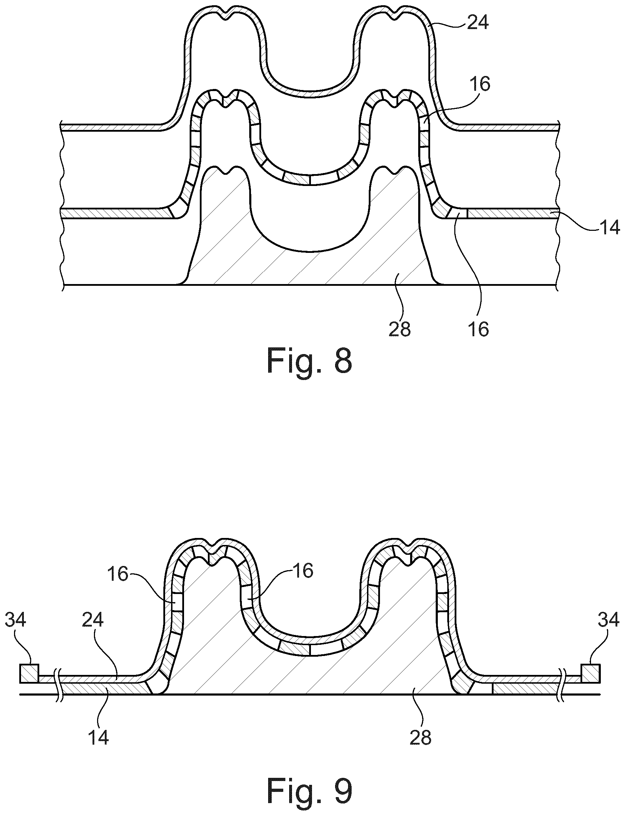

Danach, in einem dritten Schritt, wird die Dichtfolie 24 von der Funktionsfolie 14 abgenommen. Das ist in Figur 8 schematisch illustriert. Selbstverständlich kann nach erfolgtem Tiefziehen auch die Funktionsfolie 14, die während des Tiefziehens zum Schienenkörper 12 verformt wurde, vom Zahnmodell 28 abgenommen werden.

Für den Fall, dass das Folienmaterial 22 aus Figur 4 verwendet wurde, kann zum Abnehmen der Dichtfolie 24 die Verbindungsschicht 26 mittels Wasser aufgelöst werden. Wie bereits erwähnt, ist die Wasserlöslichkeit der Verbindungsschicht 26 optional.

Falls das Folienmaterial 22 gemäß Figur 5 ausgebildet ist, kann die Dichtfolie 24 mit Wasser aufgelöst und auf diese Weise von der zahnmedizinischen Funktionsfolie 14 abgenommen werden. Wie bereits erwähnt, ist die Wasserlöslichkeit der Dichtfolie 24 optional.

Im Zahnschienenkörper 12, d. h. in der tiefgezogenen Funktionsfolie 14 ist bevorzugt wenigstens ein Teil der Durchgangsöffnungen 16 patientenindividuell positioniert und/oder gestaltet. Das bedeutet, dass wenigstens ein Teil der Durchgangsöffnungen 16 für denjenigen Patienten spezifisch ist, für den die Zahnschiene 10 hergestellt wird. Für einen anderen Patienten wären diese Durchgangsöffnungen 16 anders positioniert und/oder anders gestaltet.

In einem Fall, in dem Durchgangsöffnungen 16 stets einen im Wesentlichen kreisförmigen Querschnitt haben, kann somit ein Mittelpunkt des kreisförmigen Querschnitts sowie ein Durchmesser des kreisförmigen Querschnitts patientenindividuell festgelegt werden.

Darüber hinaus sind im Zahnschienenkörper 12, d. h. in der tiefgezogenen Funktionsfolie 14, bevorzugt wenigstens ein Teil der Durchgangsöffnungen 16 basierend auf wenigstens einem Anatomiemerkmal positioniert und/oder gestaltet. Beispielsweise sind in diesem Zusammenhang Durchgangsöffnungen 16 auf den Zahnhöckern platziert. Dabei kann ein Querschnitt der Durchgangsöffnungen 16 abhängig von einem Größenkennwert der Zähne gewählt sein.

Es versteht sich, dass besonders bevorzugt ein Teil der Durchgangsöffnungen 16 sowohl patientenindividuell als auch basierend auf einem Anatomiemerkmal positioniert und/oder gestaltet ist.

Optional bilden ferner ein Teil der Durchgangsöffnungen 16, die nach dem Tiefziehen an einem Rand des Zahnschienenkörpers 12 angeordnet sind, eine Sollbruchstelle, entlang derer die Zahnschiene 10 von übrigen Abschnitten der Funktionsfolie 14 getrennt werden kann. Mit anderen Worten bilden diese Durchgangsöffnungen 16 eine Perforation, entlang derer die Zahnschiene 10, genauer gesagt der Zahnschienenkörper 12 aus den übrigen Abschnitten der Funktionsfolie 14 herausgebrochen werden kann.

Dabei ist die Position und Gestalt derjenigen Durchgangsöffnungen 16, die patientenindividuell angeordnet und/oder gestaltet sind hinsichtlich ihrer Position, Größe und Form zunächst in der tiefgezogenen Zahnschiene 10 festgelegt. Vereinfacht gesagt wird zunächst der Endzustand dieser Durchgangsöffnungen 16 festgelegt. Dasselbe gilt für diejenigen Durchgangsöffnungen 16, die basierend auf einem Anatomiemerkmal angeordnet und oder gestaltet sind. Ebenfalls gilt dies für diejenigen Durchgangsöffnungen 16, die Bestandteil der Sollbruchstelle sind.

Im Folienmaterial 22 in seiner Ausgangsform, d. h. vor dem Tiefziehen, sind diese Durchgangsöffnungen 16 derart verzerrt vorgesehen, dass sich nach dem Tiefziehen die zuvor festgelegte Position, Größe und Form ergibt. Genauer gesagt sind in der zahnmedizinischen Funktionsfolie 14 in ihrer Ausgangsform, d. h. vor dem Tiefziehen, die Durchgangsöffnungen 16 derart verzerrt vorgesehen, dass sich nach dem Tiefziehen die zuvor festgelegte Position, Größe und Form ergibt. Dabei können die verzerrten Positionen, Größen und Formen der Durchgangsöffnungen 16 mittels einer Tiefzieh-Simulation ermittelt werden.

Die Figur 6 zeigt in diesem Zusammenhang lediglich einige repräsentative Durchgangsöffnungen 16.

Für den Fall, dass die mittels des Verfahrens zum Herstellen der Zahnschiene 10 produzierte Zahnschiene 10 gemäß Figuren 2 und 3 ausgebildet ist, d. h. eine Aufnahmetasche 20 für einen Intraoralsensor 18 aufweist, wird diese Aufnahmetasche 20, allgemeiner gesprochen eine Sensorschnittstelle 32, ebenfalls während des Tiefziehens aus Funktionsfolie 14 hergestellt. Dabei ist wenigstens ein Teil der Durchgangsöffnungen 16 im Bereich der Sensorschnittstelle 32 angeordnet, sodass Speichelfluss im Bereich des Intraoralsensors 18 erfolgen kann.

Zusammengefasst bedeutet das für das Folienmaterial 22 in seiner Ausgangsform, d. h. vor dem Tiefziehen, dass ein Teil der Mehrzahl an Durchgangsöffnungen 16 in einem Bereich einer Sensorschnittstelle 32 angeordnet ist, ein Teil der Mehrzahl an Durchgangsöffnungen 16 patientenindividuell positioniert und/oder gestaltet ist, und ein Teil der Mehrzahl an Durchgangsöffnungen 16 basierend auf wenigstens einem Anatomiemerkmal positioniert und/oder gestaltet ist.

Ein derartiges Folienmaterial 22 kann als Ausgangsmaterial für das Verfahren zum Herstellen der Zahnschiene 10 bereitgestellt werden (siehe nochmals Figur 6).

Gemäß einer Option umfasst das Verfahren zusätzlich das Einbringen der Durchgangsöffnungen 16. In einem Verfahren gemäß dieser Option wird also die zahnmedizinische Funktionsfolie 14 in einem Zustand bereitgestellt, in dem diese keine Durchgangsöffnungen 16 aufweist. In einem dem ersten Verfahrensschritt vorgelagerten Schritt werden dann die Durchgangsöffnungen 16, die wie bereits beschrieben patientenindividuell angeordnet und/oder gestaltet und/oder basierend auf wenigstens einem Anatomiemerkmal angeordnet und/oder gestaltet sein können, in die Funktionsfolie 14 eingebracht. Dies kann mittels eines Laserstrahls erfolgen.

Erst danach werden die Durchgangsöffnungen 16 mittels der Dichtfolie 24 luftdicht verschlossen, sodass anschließend, wie bereits beschrieben, die Funktionsfolie 14 und die Dichtfolie 24 zusammen tiefgezogen werden können.

Das Verfahren zum Herstellen der Zahnschiene 10 kann auch gemäß einer Alternative ausgeführt werden. Dies ist in Figur 9 illustriert.

Dabei wird im Folgenden lediglich auf die Unterschiede zum bereits erläuterten Verfahren eingegangen.

Der Kern-Unterschied besteht darin, dass bei der Verfahrensvariante die Dichtfolie 24 als Bestandteil einer Anlage zum Tiefziehen ausgebildet ist. In diesem Zusammenhang ist die Dichtfolie 24 auf einen Rahmen 34 gespannt.

Als Ausgangsmaterial für die Verfahrensvariante wird also lediglich die zahnmedizinische Funktionsfolie 14 mit den Durchgangsöffnungen 16 verwendet. Diese zahnmedizinische Funktionsfolie 14 wird auf das Zahnmodell 28 aufgelegt und es wird dann die Dichtfolie 24, beispielsweise durch Bewegen des Rahmens 34, auf die Funktionsfolie 14 aufgelegt. Dadurch werden die Durchgangsöffnungen 16 luftdicht verschlossen und es kann die Funktionsfolie 14 gemeinsam mit der Dichtfolie 24 tiefgezogen werden. Danach wird die Dichtfolie 24 von der Funktionsfolie 14, die dann den Zahnschienenkörper 12 bildet, abgenommen, indem beispielsweise der Rahmen 34 bewegt wird.

### Bezugszeichenliste

- 10: Zahnschiene
- 12: Zahnschienenkörper
- 14: zahnmedizinische Funktionsfolie
- 16: Durchgangsöffnung
- 18: Intraoralsensor
- 20: Aufnahmetasche
- 22: Folienmaterial
- 24: Dichtfolie
- 26: Verbindungsschicht
- 28: Zahnmodell
- 30: Zahnschienenbaugruppe
- 32: Sensorschnittstelle
- 34: Rahmen

## Patentansprüche

1. Verfahren zum Herstellen einer Zahnschiene (10), umfassend
- Bereitstellen einer zahnmedizinischen Funktionsfolie (14), wobei in der Funktionsfolie (14) eine Mehrzahl an Durchgangsöffnungen (16) für Speichel und/oder Nahrungspartikel vorhanden ist,
- luftdichtes Verschließen der Mehrzahl an Durchgangsöffnungen (16) mittels einer Dichtfolie (24),
- gemeinsames Tiefziehen der Funktionsfolie (14) und der Dichtfolie (24) entlang eines Zahnmodells (28), und
- anschließendes Abnehmen der Dichtfolie (24) von der Funktionsfolie (14).

2. Verfahren nach Anspruch 1, wobei das luftdichte Verschließen der Mehrzahl an Durchgangsöffnungen (16) mittels der Dichtfolie (24)
ein Bereitstellen der Dichtfolie (24) und der Funktionsfolie (14) als Bestandteile eines mehrschichtigen Folienmaterials (22) umfasst, oder ein Auflegen der Dichtfolie (24) auf die Funktionsfolie (14) umfasst.

3. Verfahren nach Anspruch 1 oder 2, wobei die Funktionsfolie (14) und die Dichtfolie (24) direkt aneinander anliegen oder wobei die Funktionsfolie (14) und die Dichtfolie (24) mittels einer Verbindungsschicht (26) verbunden sind.

4. Verfahren nach einem der vorhergehenden Ansprüche, wobei die Dichtfolie (24) oder die Verbindungsschicht (26) wasserlöslich ist und das Abnehmen der Dichtfolie (24) von der Funktionsfolie (14) ein Auflösen der wasserlöslichen Dichtfolie (24) oder der wasserlöslichen Verbindungsschicht (26) umfasst.

5. Verfahren nach einem der vorhergehenden Ansprüche, ferner umfassend Herstellen einer Sensorschnittstelle (32) in der Funktionsfolie (14), wobei wenigstens ein Teil der Mehrzahl an Durchgangsöffnungen (16) im Bereich der Sensorschnittstelle (32) angeordnet ist.

6. Verfahren nach einem der vorhergehenden Ansprüche, wobei wenigstens ein Teil der Mehrzahl an Durchgangsöffnungen (16) in der Funktionsfolie (14) patientenindividuell positioniert und/oder gestaltet ist.

7. Verfahren nach einem der vorhergehenden Ansprüche, wobei wenigstens ein Teil der Mehrzahl an Durchgangsöffnungen (16) in der Funktionsfolie (14) basierend auf wenigstens einem Anatomiemerkmal positioniert und/oder gestaltet ist.

8. Verfahren nach einem der vorhergehenden Ansprüche, wobei wenigstens ein Teil der Mehrzahl an Durchgangsöffnungen (16) in der Funktionsfolie eine Sollbruchstelle zum Trennen der Zahnschiene (10) von überschüssiger Funktionsfolie (14) bildet.

9. Verfahren nach einem der vorhergehenden Ansprüche, wobei wenigstens ein Teil der Mehrzahl an Durchgangsöffnungen (16) in der Funktionsfolie (14) basierend auf einer in der tiefgezogenen Zahnschiene (10) festgelegten Position, Größe und/oder Form in der Funktionsfolie (14) derart verzerrt ist, dass sich nach dem Tiefziehen die festgelegte Position, Größe und/oder Form ergibt.

10. Verfahren nach einem der vorhergehenden Ansprüche, wobei das Bereitstellen der zahnmedizinischen Funktionsfolie (14) umfasst:
- Bereitstellen der Funktionsfolie (14) in einem unperforierten Zustand, und
- Einbringen der Mehrzahl an Durchgangsöffnungen (16) in die Funktionsfolie (14).

11. Zahnschiene (10), erhalten mittels eines Verfahrens nach einem der vorhergehenden Ansprüche.

12. Zahnschienenbaugruppe (30), umfassend einen Zahnschienenkörper (12), der aus einer zahnmedizinischen Funktionsfolie (14) gebildet ist, die eine Mehrzahl an Durchgangsöffnungen (16) für Speichel und/oder Nahrungspartikel aufweist, wobei die Durchgangsöffnungen (16) mittels einer Dichtfolie (24) luftdicht verschlossen sind.

13. Folienmaterial (22) zum Herstellen einer Zahnschiene (10), wobei das Folienmaterial (22) eine zahnmedizinische Funktionsfolie (14), in der eine Mehrzahl an Durchgangsöffnungen (16) für Speichel und/oder Nahrungspartikel vorgesehen ist, und eine Dichtfolie (24) umfasst, welche die Mehrzahl an Durchgangsöffnungen (16) luftdicht verschließt.

14. Folienmaterial (22) nach Anspruch 13, wobei die Funktionsfolie (14) und die Dichtfolie (24) direkt aneinander anliegen oder wobei die Funktionsfolie (14) und die Dichtfolie (24) mittels einer Verbindungsschicht (26) verbunden sind.

15. Folienmaterial (22) nach Anspruch 13 oder 14, wobei
wenigstens ein Teil der Mehrzahl an Durchgangsöffnungen (16) in einem Bereich einer Sensorschnittstelle (32) für einen Sensor (18) zur Detektion eines Biomarkers im Speichel angeordnet ist, und/oder
wobei wenigstens ein Teil der Mehrzahl an Durchgangsöffnungen (16) patientenindividuell positioniert und/oder gestaltet ist, und/oder wobei wenigstens ein Teil der Mehrzahl an Durchgangsöffnungen (16) basierend auf wenigstens einem Anatomiemerkmal positioniert und/oder gestaltet ist.
